# EUROPEAN PATENT APPLICATION

(11) **EP 4 843 500 A1**
(43) Date of publication of application: **25.11.2026**
(21) Application number: 25178616.6
(22) Date of filing: 23.05.2025
(51) Int. Cl.: A61B 5/16, A61B 5/18, A61B 5/00

(54) **IMPAIRMENT DETECTION BASED ON EYE-TRACKING METRICS**

(71) Applicant: Smart Eye AB, 413 29 Göteborg (SE)
(72) Inventor: ZEMBLYS, Raimondas, 417 53 Göteborg (SE)
(74) Representative: Lind Edlund Kenamets Intellectual Property AB

(57) **Abstract**

The present disclosure relates to a method and a system for predicting impairment of an occupant of a vehicle, wherein the system comprises a controller module comprising one or more processors configured to obtain, by a plurality of imaging sensors arranged on board the vehicle, a first set of measurements capturing one or more eye-movement patterns of the occupant. The controller module s further configured to generate a gaze pattern profile for the occupant and determine if the generated gaze pattern profile corresponds to an eye-movement pattern associated with reading textual information. Further, the controller module is configured to determine, based at least partly on the generated gaze pattern corresponding to the eye-movement pattern associated with reading textual information, a probability and the level of impairment of the occupant.

## Description

### FIELD OF THE INVENTION

The present disclosure generally relates to predicting impairment of an occupant of a vehicle based on eye-tracking metrics. More specifically, aspects and embodiments herein relate to predicting impairment of the occupant of the vehicle based on eye movement patterns associated with reading textual information by the occupant.

### BACKGROUND OF THE INVENTION

Driving in an impaired state, e.g., under the influence of alcohol or drugs remains one of the leading causes of traffic accidents and fatalities worldwide. Conventional countermeasures rely on chemical analysis of a driver's breath, most commonly via portable breathalyzers or ignition-interlock breath-test devices that demand a satisfactory alcohol reading before the vehicle can be started. Blood-draw and laboratory assays of blood-alcohol concentration (BAC) or toxicology panels provide a gold standard, yet they are invasive, require trained personnel, and cannot be deployed continuously or at the roadside. Other established techniques include roadside field-sobriety tests administered by law-enforcement officers, which often combine balance tasks with handheld breath tests to confirm intoxication levels.

Although effective in some scenarios, these approaches have notable drawbacks. For instance, breathalyzers require active user compliance, can be circumvented or yield false readings if improperly calibrated; blood tests are slow, inconvenient, and unsuitable for real-time, in-vehicle screening; and field-sobriety testing is time-consuming and impractical for automated monitoring.

Recent research has investigated eye-tracking systems that analyze eye movements of the occupants of the vehicle as proxies for detecting cognitive impairment under the influence of alcohol or drugs. However, prevailing prototypes typically subject the occupants to multi-stage cognitive test sequences, such as following moving visual stimuli, directing their gaze at certain directions, executing rapid gaze-shift tasks, or actively responding to on-screen commands that demand precise timing and explicit user input and involvement to perform visual cognitive tasks such as pattern recognition and the like. This complexity increases the risk of user confusion or incorrect execution (e.g. looking at the wrong target, or missing a cue, etc.), compromises reliability under stress or fatigue, and lengthens the overall screening procedure, consequently also leading to user frustration.

Accordingly, there remains a clear need in the field of occupant impairment detection by eye tracking systems for more accurate, reliable and user-friendly solutions that minimize test time, reduce cognitive load, and lower system complexity while enabling drivers to assume, regain or retain safe control of the vehicle.

### SUMMARY OF THE INVENTION

It is an object of the present invention to provide systems and methods for solving or at least alleviating some of the above-mentioned drawbacks and problems of the prior art.

These and other objects may be achieved by the impairment detection system and the method of detecting impairment of an occupant of a vehicle as defined in the appended independent claims herein. Various embodiments of the present invention have been defined in the dependent claims.

The term exemplary is in the following to be interpreted as serving as an example, example implementation, instance, scenario or illustration.

According to a first aspect of the present invention, there is provided a computer-implemented method for predicting impairment of an occupant of a vehicle. The method comprises obtaining, by a plurality of imaging sensors arranged on board the vehicle, a first set of measurements capturing one or more eye-movement patterns of the occupant. The method further comprises generating, based on the obtained first set of measurements, a gaze pattern profile for the occupant. In several embodiments the gaze pattern profile may be associated with reading textual information by the occupant. Accordingly, the method further comprises determining if the generated gaze pattern profile corresponds to an eye-movement pattern associated with reading textual information. When it is determined that the generated gaze pattern profile corresponds to the eye-movement pattern associated with reading textual information, the method further comprises determining, based at least partly on the generated gaze pattern corresponding to the eye-movement pattern associated with reading textual information, a probability and the level of impairment of the occupant. Generating the gaze pattern profile for the occupant comprises determining a characteristic sequence of eye-movement metrics including a sequence of eye fixations and/or a sequence of eye saccades of one or both eyes of the occupant.

In some embodiments, the method may further comprise displaying a first textual information on a display module arranged on board the vehicle with which the occupant interacts. The method may further comprise prompting, by a first signal, the occupant of the vehicle to read the displayed first textual information. In some embodiments, the first signal may comprise any one of appearance of the textual information on the display, and an instruction text included in the first textual information encouraging the occupant to read the first textual information. The first signal may further comprise a visual and/or an auditory indicator directing attention of the occupant to read the displayed textual information.

The present invention is at least based on a realization that extracting eye-movement measures of one or both eyes of the occupant while reading textual information e.g. including a string of text of various length, and the like will be an efficient way to determine the probability and level of impairment or intoxication of the occupant, especially the driver intending to operate the vehicle. This is at least partly based on the realization that eyes follow a particular movement pattern that during reading text and thus generating an eye gaze pattern that is uniquely different from eye movements generated during performing other visual tasks such as following trajectory or movement of objects in space, looking in certain directions, and the like. More particularly, the present invention is based on the realization that the eye tracking system may be configured to continuously monitor the eye movements of the occupants, and compare the recorded eye movements to a predetermined unique gaze pattern associated with reading textual information. Upon determining that the recorded eye movements of the occupant corresponds to the predetermined reading eye movement pattern, it will be flagged that the occupant is engaged in a reading task. Accordingly, the recorded eye gaze pattern will be utilized by an impairment detection system to assess the level and probability of impairment of the occupant. According to the presented inventive concept, the need for tedious and ineffective visual, or cognitive tests designed to scrutinize the occupants' impairment are advantageously alleviated.

In several embodiments, the step of determining the characteristic sequence of eye-movement parameters included in the gaze pattern profile may comprise determining a first subset of metrics including an eye fixation duration, an eye fixation rate, an eye saccade duration, an eye saccade rate, an eye saccade amplitude, and an eye saccade peak velocity for one or both eyes of the occupant.

In some embodiments, the step of determining the characteristic sequence of eye-movement parameters included in the gaze pattern profile may further comprise determining a second subset of metrics including any one of static entropy measurements and/or transition entropy measurements of the eye fixation duration, the eye fixation position, the eye saccade duration, the eye saccade amplitude, the eye saccade peak velocity. The second subset of metrics may further include a relationship between the eye fixation duration and parameters of the subsequent eye saccade.

In some embodiments, the step of determining the characteristic sequence of eye-movement parameters included in the gaze pattern profile may further comprise determining a third subset of metrics including any one of a gaze regression, number of eye fixations and eye saccades per word comprised in the textual information e.g. the first textual information, the eye fixation position within a word comprised in the textual information e.g. the first textual information, and the first eye fixation position within a word comprised in the textual information.

In some exemplary embodiments, the method may further comprise obtaining, by the plurality of imaging sensors arranged on board the vehicle, a second set of measurements capturing at least one parameter associated with the occupant. The second set of measurements may be obtained during an event or routine of reading the textual information by the occupant. The second set of measurements may comprise any one of an eye blink duration, an eye blink closing and opening velocity, an eye blink closing and opening duration, and a head pose and/or head movements of the occupant. The method may further comprise determining the probability and the level of impairment of the occupant based further on the obtained second set of measurements.

In some embodiments, the method may further comprise upon determining that the probability and the level of impairment of the occupant falls within an acceptable range, generating a control signal in order to extend control of the vehicle to the occupant in order to operate the vehicle.

In some embodiments, the method may further comprise upon determining that the probability and the level of impairment of the occupant falls outside an acceptable range such as below a threshold value, determining that the occupant of the vehicle is in an impaired state. The method may further comprise generating a control signal in order to perform any one of deactivating an ignition system of the vehicle and preventing the occupant from turning on the ignition system, deactivating the transmission system of the vehicle, when in an autonomous driving mode, preventing an automated driving system (ADS) comprised in of the vehicle from extending control of the vehicle to the occupant, when in an autonomous driving mode, instructing the ADS to bring the vehicle to a stop, and instructing the ADS to contact emergency services for assistance.

In some example embodiments, the method may further comprise generating a second signal indicating an outcome of the determination of impairment of the occupant. When the probability and level of impairment of the occupant falls within the acceptable range, the method may further comprise prompting, by the second signal, the occupant of the vehicle to trigger an interactive control element activated on the display. The second signal may comprise any one of generating and displaying a second textual information on the display module indicative of the outcome of the determination process of the probability and the level of impairment of the occupant. The second signal may further comprise an activation of a previously deactivated interactive control element on the display. The second signal may further comprise a text message and/or a visual indicator presented on the display and/or an auditory indicator directing the occupant to trigger the interactive control element.

In some exemplary embodiments, the method may further comprise extending control of the vehicle to the occupant in order to operate the vehicle after the occupant of the vehicle has triggered the interactive control element activated on the display.

According to a second aspect, there is provided an impairment detection system for predicting impairment of an occupant of a vehicle. The system comprises a controller module comprising one or more processors. The controller module is configured to obtain, by a plurality of imaging sensors arranged on board the vehicle, a first set of measurements capturing one or more eye-movement patterns of the occupant. Further, the controller module is configured to generate, based on the obtained first set of measurements, a gaze pattern profile for the occupant. The controller module is further configured to determine if the generated gaze pattern profile corresponds to an eye movement pattern associated with reading textual information. Upon the determination, the controller module is further configured to determine, based at least partly on the generated gaze pattern corresponding to the eye-movement pattern associated with reading textual information, a probability and the level of impairment of the occupant. The controller module is further configured to generate the gaze pattern profile by determining a characteristic sequence of eye-movement metrics including a sequence of eye fixations and/or a sequence of eye saccades of one or both eyes of the occupant.

According to a third aspect of the present invention, there is provided a computer program product comprising instructions which, when the program is executed by one or more processors of a processing system, causes the processing system, to carry out the methods according to any one of the embodiments of the method according to the first aspect.

According to a fourth aspect of the present invention, there is provided a computer-readable storage medium, such as a non-transitory computer-readable storage medium, storing one or more programs configured to be executed by one or more processors of a processing system, the one or more programs comprising instructions to carry out the methods according to any one of the embodiments of the method according to the first aspect. The control module executing the one or more programs may be a control module of an eye tracking system, an impairment detection system, or a control system of the vehicle, or a combination thereof operating in conjunction with each other.

Further embodiments of the different aspects are defined in the dependent claims. It is to be noted that all the embodiments, elements, features and advantages associated with the first aspect also analogously apply to the second, third, and fourth aspects of the present disclosure and *vice versa.*

These and other features and advantages of the present disclosure will be further explained in the following detailed description.

### BRIEF DESCRIPTION OF THE DRAWINGS

This and other aspects of the present invention will now be described in more detail, with reference to the appended drawings showing embodiments of the present invention. The drawings are only schematic and the relative dimensions of some structures and layers may be exaggerated and not drawn to scale. Rather the dimensions may be adapted for illustrational clarity and to facilitate understanding.
Fig. 1 shows a schematic illustration of an interior of a vehicle comprising an eye tracking system and an impairment detection system according to some embodiments of the present disclosure.
Fig. 2 shows a schematic block diagram illustration of a control system of the vehicle according to some embodiments of the present disclosure.
Fig. 3 shows a schematic illustration of a display module aboard the vehicle according to some embodiments of the present disclosure.
Fig. 4 shows a schematic flowchart illustrating a method in accordance with several embodiments of the present disclosure.

### DETAILED DESCRIPTION

The following disclosure presents various embodiments or examples for implementing different aspects of the provided subject matter. Specific examples of implementing the method are described to simplify the disclosure. These examples are not intended to be limiting. Additionally, reference numerals and/or letters may be repeated in various examples for simplicity and clarity, without implying a specific or limiting relationship between the different embodiments and/or configurations discussed, unless stated otherwise.

It is further to be noted that terms such as "first" and "second" etc. with reference to elements or steps may be used herein as labels to facilitate distinguishing between different elements and need not necessarily imply that such elements or steps are arranged or performed in that particular order, unless stated otherwise.

It is also to be understood that the terminology used herein is for the purpose of describing particular embodiments only and is not intended to be limiting. It should be noted that, as used in the specification and the appended claim, the articles "a", "an", "the", and "said" are intended to mean that there are one or more of the elements unless the context clearly dictates otherwise. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative.

Those skilled in the art will appreciate that the steps, services and functions explained herein may be implemented using individual hardware circuitry, using software functioning in conjunction with a programmed microprocessor or general purpose computer, using one or more Application Specific Integrated Circuits (ASICs) and/or using one or more Digital Signal Processors (DSPs). It will also be appreciated that when the present disclosure is described in terms of a method, it may also be embodied in one or more processors and one or more memories coupled to the one or more processors, wherein the one or more memories store one or more programs that perform the steps, services and functions disclosed herein when executed by the one or more processors.

In the following description of exemplary embodiments, the same reference numerals denote the same or similar components. Even though the following disclosure mainly discusses vehicles in the form of cars, the skilled reader readily realizes that the teachings discussed herein are applicable to other forms of vehicles such as trucks, buses, construction equipment or the like operated by human operators or having various degrees of automated driving features.

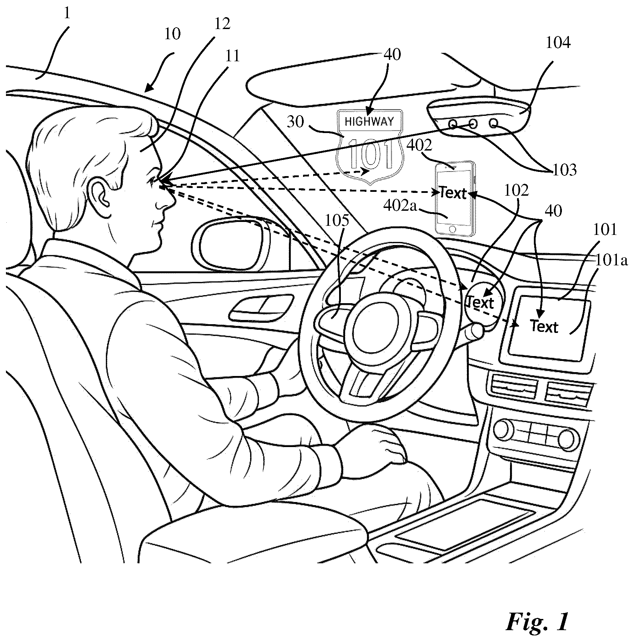

Fig. 1a shows an occupant 10 of a vehicle 1. In this example the occupant is a driver 10 sitting in a driver's seat of the vehicle 1. The occupant 10 is shown to face an infotainment system 101 and instrument cluster 102 of the vehicle 1. The vehicle 1 comprises an eye tracking system 300. In some examples, the eye tracking system 300 may have a sampling rate of up to 60 Hz, or up to 120 Hz or more preferably 60 Hz or higher, e.g., at least 120 Hz. Furthermore, the vehicle 1 comprises a plurality of imaging sensors 103 arranged on board the vehicle 1 for capturing image data and/or video data of the interior of the vehicle 1 including image data and/or video data from one or more occupants such as occupant 10 present inside the vehicle 1. The plurality of imaging sensors 103 may be camera sensors 103. The camera sensors may be any type of suitable camera sensors 103 for instance visible light camera sensors 103, infrared or night vision or thermal camera sensors 103 and the like. The one or more imaging sensors 103 may be comprised as a part of the eye-tracking system, or may be included in the multi-purpose sensor devices of the vehicle 1 utilized by the eye-tracking system of the vehicle 1 for eye-tracking applications and functionalities which will be described herein.

The one or more imaging sensors 103 may be mounted on different parts of the interior of the vehicle 1. For instance, in Fig.1 the one or more imaging sensors are mounted in front of the occupant 10, on or integrated within the rearview mirror 104. Alternatively, or additionally, the one or more imaging sensor devices 103 may be mounted on the dashboard, as a part of the infotainment system 101, as a part of the instrument cluster 102, on the steering wheel column 105, or fixed to the ceiling or any other part of the vehicle 1 that enable capturing image and/or video data of the occupants 10 of the vehicle. Regardless of the spatial position of the one or more imaging sensors 103, the mounted imaging sensors 103 have a clear view of the upper body of vehicle occupants 10 intended to be monitored. Particularly, the one or more imaging sensors 103 are configured to capture image and/or video data of the one or both eyes 11, as well as head pose of the head 12 of the occupants 10. Typically, imaging sensors 103 mounted in the front of the vehicle, e.g. mounted on the rearview mirror 104, are able to monitor at least the driver 10 and the front passenger (not specifically shown). The imaging sensors 103 may typically be arranged at a predetermined distance from the occupants 10. In this example, the range may vary from 40 cm to 120 cm, or may be at "arm length", i.e. around 60-70 cm or similar, depending on the system requirements.

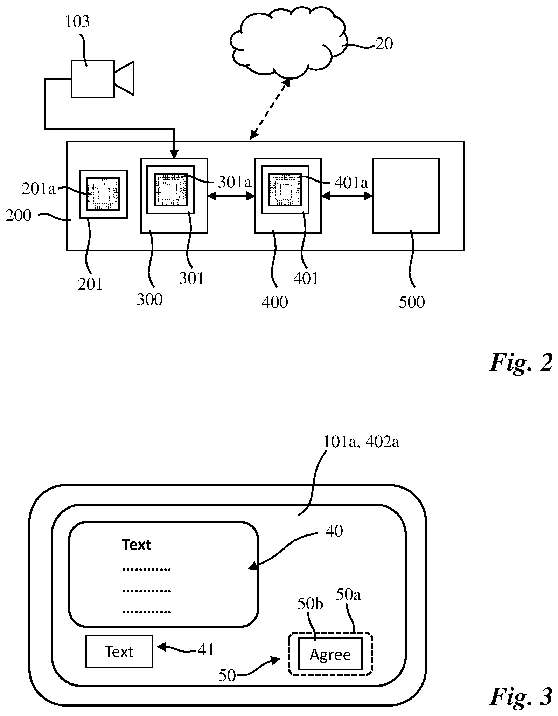

The one or more imaging sensors 103 are operatively connected to the eye-tracking system, and more specifically to a controller module 301 or processing system 301 of the eye-tracking system 300 as shown in the block diagram illustration in Fig. 2. The controller module 301 may comprise one or more processors or processing circuitry 301a for performing different operations such as data acquisition, data processing, running various data analysis algorithms or models, etc. The system tracking system 300 or the controller module 301 may optionally include designated operational modules (not specifically shown) for performing designated operations such as image acquisition module, image analysis and data extraction module, communication module for receiving and transferring data among multiple modules and entities, and so on.

The vehicle 1 of Fig. 1 comprises a system 400 for determining and predicting impairment of one or more occupants of the vehicle 1. The impairment may occur because of substance abuse, e.g., being under influence of alcohol and/or any kind of psychoactive drugs or medication affecting occupant's mental, behavioral, cognitive or motor functions. In some examples the impairment may be due to drowsiness or extreme tiredness equally diminishing the normal cognitive and mental functionality of the occupants 10.

In some embodiments, the occupant 10 that will be inspected by the impairment detection system 400 is the occupant 10 sitting behind the steering wheel of the vehicle intending to drive or otherwise operate the vehicle 1. In some examples, the occupant 10 may be a passenger of the vehicle 1. In some example embodiments, the system 400 may be a separate entity or may be at least partly implemented by the eye-tracking system 300 and/or the control system 200 of the vehicle 1. In some embodiments, the impairment detection system 400 may comprise the eye-tracking system 300 or an eye-tracking module to perform eye tracking operations. Alternatively, or additionally the impairment detection system 400 and functionalities may be included in the ADS architecture of the vehicle 1. In any case, the impairment detection system 400 is configured to be in operative connection with the eye-tracking system 300. The impairment detection system 400 may comprise a controller module 401 comprising one or more processors 401a configured to carry out various functionalities presented herein. The impairment detection system 400 or the controller module 401 may optionally include designated operational modules (not specifically shown) for performing designated operations such as data analysis modules, data comparison modules, image analysis and data extraction modules, modules implementing various algorithms such as machine learning (ML) or neural network (NN) algorithms and the like.

In some example implementations, the impairment detection system 400 may be implemented as an standalone solution such as in a separate instrument such as a handheld module 402 that could be installed and operatively connected to the control system 200 of the vehicle 1. The control system 200 may comprise a controller module 201 comprising one or more processors 201a configured to carry out various functionalities presented herein.

Some of the various embodiments of the methods presented herein may at least partly be performed by the controller module 301 of the eye-tracking system 300 in conjunction with the controller module 401of the impairment detection system 400. Alternatively, or in addition the methods may be partly or entirely be performed by the control system 200 or processing system 200 of the vehicle 1. The control system or processing unit of the vehicle may be in operative connection with the controller module 301 of the eye-tracking system 300. In some cases, the eye-tracking system 300 and the controller module 301 thereof may be standalone units or may be comprised in the control system 200 or processing unit 200 of the vehicle. For the sake of simplicity only, in the rest of this specification, various functions and operations will be explained as being carried out by the impairment detection system 400.

In some example implementations, the vehicle 1 may comprise an Automated Driving System (ADS). In the present context, the ADS comprises both advanced driver-assistance systems (ADAS) and Autonomous Driving (AD) systems. ADS herein corresponds to all of the different levels of automation as for example defined by the SAE J3016 levels (0 - 5) of driving automation, and in particular for level 4 and 5. The ADS systems are capable of employing their ADAS features including integrated sensor systems, etc. for surveillance of the surrounding environment of the vehicle, displaying information such as warning signals etc. to the occupants of the vehicle. Further, the ADS systems may comprise AD systems for autonomously operating and controlling one or more of acceleration, steering, and braking of the vehicle 1. As mentioned above, the vehicle 1 comprises a control system which may be a separate entity or may be a part of the overall ADS architecture of the vehicle, and may accordingly be a module or component of the ADS. In some embodiments, the eye-tracking system 300 or the impairment detection system 400 may be a separate entity or may be a part of the overall ADS architecture 500 of the vehicle, and in any case be in operative connection with the ADS architecture and the control system 200 of the vehicle 1. In Fig. 2, the eye-tracking system 300, the impairment detection system 400, and the ADS system 500 are shown to be comprised in the overall control system 200 of the vehicle 1 for the sake of illustration. As also illustrated in Fig. 2, parts of the described solutions herein may be implemented either in the vehicle 1, in a system located external to the vehicle, or a combination thereof. For instance, parts of the algorithms and models may be stored and executed in a remote server in communication with the vehicle 1, a so-called cloud solution 20.

In the present context "impairment" is to be understood as a state that corresponds to a degree of drunkenness, or be under influence of the drugs, narcotics, or certain medications that will distort the normal cognitive and functioning of the occupants. When the occupant 10 is the driver 10 of the vehicle 1 intending to operate and drive the vehicle 1, being in a state of impairment will mean that the driver 10 is not in conditions suitable for safely operating a motor vehicle 1 in traffic and might pose a danger to themselves or other vehicles or pedestrians in traffic.

The impairment detection system 400 is configured to obtain e.g. through the eye tracking system 300, various patterns of eye movements and gaze measurement parameters of one or both eyes of the occupants 10 of the vehicle 1 recorded by the one or more imaging sensor devices 103 on board the vehicle 1. The impairment detection system 400 is further configured to extract various gaze parameters and characteristic sequences of eye movements in order to generate one or more gaze pattern profiles for each occupant 10 of the vehicle 1. In several embodiments, the impairment detection system 400 may be configured in operative connection with the control system 200 of the vehicle 1 such that it may generate and transmit control signals to the control system 200 of the vehicle 1. The eye tracking system 300 and the impairment detection system 400 may also be arranged to receive control signals and/or instructions from the control system 200 of the vehicle 1. In several exemplary embodiments, the impairment detection system 400 may generate control signals to control at least one function of the vehicle including core power-train elements such as the ignition system, driver monitoring systems (DSM), operation of the engine of the vehicle 1, operation of the gearbox and power electronics, chassis-dynamics devices like braking, steering assistance, battery management systems, infotainment, telematics and instrument clusters, safety, convenience, and autonomous features such as ADS features to name a few.

The occupant impairment detection system 400, according to several embodiments herein is configured to continuously monitor the occupants 10 of the vehicle 1 by acquisition of gaze patterns through the eye tracking 300 in order to discern the probability and level impairment e.g. drunkenness of the occupants 10. More particularly, the impairment detection system 400 is configured to determine the probability and level of occupant impairment through detection of eye gaze patterns that are specific for reading textual information. Accordingly, the eye tracking system 300 will continuously monitor and record eye movements of the occupants 10 of the vehicle 1 during the time they are present in the vehicle 1. This applies both to the initial moment when occupants 10 enter and settle into their seats, before the engine of the vehicle 1 is ever started and put in motion and/or to any later point in the journey during the operation and driving the vehicle, after the occupants 10 have already been aboard for some time.

The present inventor has realized that extracting eye-movement measures of one or both eyes of the occupant 10 while reading textual information 40 e.g. including a string of text 40 of various length, and the like will be an efficient way to determine the probability and level of impairment or intoxication of the occupant 10, especially the driver 10 intending to operate the vehicle 1. This is at least partly based on the realization that eyes follow a particular movement pattern that during reading text and thus generating an eye gaze pattern that is uniquely different from eye movements generated during performing other visual tasks such as following trajectory or movement of objects in space, looking in certain directions, and the like. Stated differently, the impairment detection system is adapted to constantly look for specific sequences, order and timing of eye movement patterns of the occupant 10, and to assess their correspondence to predetermined sequences, order and timing of eye fixations and saccades of a reading pattern.

The recorded eye-movement measures associated with reading textual information will subsequently be used as input to algorithms and models such as ML or artificial intelligence (AI) models that are configured to predict impairment probability and impairment level of the reader.

Such predictions can then be used as an initial guess of the driver's readiness to drive the vehicle 1, assume control of the vehicle 1 from an ADS system during an autonomous operation of the vehicle 1 by the ADS, or to be provided to other DMS to further examine and establish the state of the occupant 10 based on other impairment metrics. More particularly, the present invention is based on the realization that the eye tracking system 300 may be configured to continuously monitor the eye movements of the occupants 10, and compare the recorded eye movements to a predetermined unique gaze pattern associated with reading textual information. Upon determining that the recorded eye movements of the occupant 10 corresponds to the predetermined reading eye movement pattern, it will be flagged that the occupant 10 is engaged in a reading task. Accordingly, the recorded eye gaze pattern will be utilized by the impairment detection system 400 to assess the level and probability of impairment of the occupant 10.

In more detail, the impairment detection system 400 is configured to obtain by the plurality of imaging sensors arranged on board the vehicle 1 e.g. through the eye tracking system 300, a first set of measurements capturing one or more eye-movement patterns of the occupant 10. The impairment detection system 400 will continuously generate a gaze pattern profile for the occupant 10 based on the obtained first set of measurements. It should also be appreciated that the eye movement measurements obtained by the eye tracking system 300 may be utilized to perform actions related to any other applications and by any other systems or modules aboard the vehicle 1 than the impairment detection system 400 in parallel with impairment detection or at any other point in time.

Moving on, the impairment detection system 400 is further configured to determine if the generated gaze pattern profile corresponds to the eye-movement patterns associated with reading textual information. Upon such determination being established, the impairment detection system 400 will proceed to determine, based at least partly on the generated gaze pattern, the probability and the level of impairment of the occupant 10. The gaze pattern profile comprises a characteristic sequence of eye-movement metrics including a sequence of eye fixations. Alternatively or additionally, the characteristic sequence of eye-movement metrics include a sequence of eye saccades of one or both eyes of the occupant 10. An eye fixation in the present context is to be understood as a temporal interval during which the eye's line of sight (visual axis) remains relatively stable on a limited region of the visual field, allowing the responsible parts of the eye such as fovea to acquire high-resolution information. An eye saccade in the present context is to be understood as a rapid, ballistic eye movement that repositions the fovea from one point of interest to another, bridging areas between fixations with minimal visual processing.

By the characteristic sequence of eye-movement metrics in the present context it is meant a particular ordered time series of multi-dimensional eye-tracking features including eye fixations, and eye saccades having a particular sequence, timing and spatial distribution. These characteristic sequences of the eye-movement metrics form task-specific, distinct, recognizable patterns that are tuned to the task at hand such as reading a text, visual scanning, free viewing, driving and navigation, hand-eye coordination, and the like. These specific gaze patterns are statistically stable for a given person, task, or cognitive state and therefore are usable as a discriminative signature to determine the task that the occupant 10 is performing. Thus, based on the acquired characteristic sequence of eye-movement metrics differences between reading textual information or simply skimming through the textual information can be distinguished and recorded. Furthermore, by acquiring the sequences of eye-movement features, exact ordering and timing of evens are recorded to accurately distinguish reading patterns by the occupants 10.

Accordingly, the systems and methods presented herein will continuously execute reading pattern recognition by comparing the acquired sequences of the eye-movement patterns to the distinct, task-dependent sequences of the eye movement metrics in order to promptly detect and identify instances of reading behavior by the occupant 10. Therefore, when it is established that the occupant 10 is reading a text e.g., the text 40 shown in Fig. 1, the systems and methods presented herein execute eye movement features from the recorded gaze profile associated with the reading pattern and feed this information to the designated algorithms and models for determination of occupant impairment. The text 40 may be shown on a display 101a of the infotainment system 101, on the instrument cluster 102, or on an example sign arrange in the surrounding environment of the vehicle 1 such as a roadside traffic sign 30 positioned along the travel path of the vehicle 1, or any text that may enter to the field of vision of the occupant 10 while being present inside the vehicle 1, including while driving the vehicle 1.

According to the inventive concept, the occupant 10 of the vehicle is not required to go through a visual or cognitive test session by performing specific tasks or by following specific instructions ordering the occupant 10 to complete several visual tasks, play interactive games, intentionally move their gaze to scan a specific spatial area, and so on during the test session. These test sessions are often times confusing, require the occupants to repeat multiple steps, and simply interfere negatively with the pleasant experience of driving.

According to the presented inventive concept, the need for such tedious and ineffective visual, cognitive tests designed to scrutinize the occupants' impairment and establish their ability to operate the vehicle 1 are advantageously alleviated. Instead, the occupants 10 of the vehicle 1 will experience a natural interaction with the instruments, infotainment system and instrument cluster in the vehicle 1 without going through a specific test session designed to identify signs of alcohol or drug intoxication. This way the occupants 10 e.g. the driver intending to start the vehicle 1 or to assume control of the vehicle from and ADS system may not even be aware that they need to pass a cognitive test or to perform visual tasks or even actively read a text intended to be used to run evaluation of their impairment state. This way any text that happens to appear somewhere within the field of vision of the occupant 10, e.g., a song name 40 in the infotainment system 101, a text 40 on a display 402a of the handheld instrument 402 aboard the vehicle 1, a warning message 40 in the instrument cluster 102 indicating "it is likely slippery outside", or even a text 40 outside the vehicle 1 such as the text 40 on billboards, other vehicles, the traffic signs 30, etc. that the occupants 10 might encounter.

**In** some example implementations, the impairment detection system 400 may be further configured to display a first textual information 40 on a display module arranged on board the vehicle 1 with which the occupant interacts. For instance, the display module may be the display 101a of the infotainment system 101 or the instrument cluster 102, or the display 402a of the handheld instrument 402 arranged inside the vehicle 1. The impairment detection system 400 may be further configured to prompt the occupant 10 of the vehicle 1 to read the displayed first textual information 40. The occupant 10 may be prompted into doing so by a first signal generated by the impairment system 400 either through the instruments 101, 102 of the vehicle 1 itself, or through the external instrument 402 installed in the vehicle 1. In some examples, the first signal may comprise any appearance of the textual information 40 on the display 101a, 402a. This way appearance of the text 40 on the display will attract the attention of the occupant 10 to read the text 40. Alternatively or additionally, the first signal may comprise an instruction text included in the first textual information 40 encouraging the occupant 10 to read the first textual information 40. For instance, the instruction text may simply indicate "please read the following statement prior to proceeding" or similar natural text. The instruction text may have a different style, font, font size or color to grab the attention of the occupant 10. In further examples, the first signal may additionally or alternatively comprise a visual indicator directing attention of the occupant 10 to read the displayed textual information 40. For instance, the textual information, or an indicator light signal may flash on the display module 101a, 402a. Even further, additionally or alternatively, the first signal may further comprise an auditory signal, such as a voice instructor or a voice command prompting the occupant 10 to direct their gaze toward the displayed text 40 and to perform the action of reading. It is obvious that other example signals, e.g., tactile or vibration signals to direct the occupant 10 to read the displayed text 40 are also contemplated.

Accordingly, in different examples discussed herein, when the occupant 10 such as the driver 10 enters the vehicle and attempts to start the car engine and/or attempts to take over automatic driving from the ADS during an autonomous driving occasion, natural text 40 may be displayed on the display modules 101a, 402a. Then again, the interaction of the driver 10 with the displayed text is intended to be a natural experience for the occupant 10 without subjecting the occupant 10 to a complex cognitive and visual test session. The displayed text 40 may thus be a text that the occupants 10 are used to normally encounter when trying to operate any other vehicle. For instance, the displayed text 40 may be a disclaimer text, terms of service text, a greeting text, a car brand slogan, a confirmation text indicating that the driver is about to take over control of the vehicle, etc. The point here is that the displayed text 40 is natural text that could be in any language used to display messages, information or similar. The text 40 may be of any length, format or structure. Naturally, even when the occupant 10 is prompted by the first signal to read the displayed text 40, the experience of the occupant 10 will be a natural interaction with the on-board systems of the vehicle 1.

Accordingly, the impairment detection system 400 utilizes the generated gaze pattern profile of the occupant 10 including the characteristic sequence of fixations and/or saccades from left to right, right to left, or top to down. The system 400 will also use the generated gaze pattern profile to establish its correspondence to the reading pattern characteristics. In some example embodiments, after the system 400 has established that a reading task is performed, the impairment detection system 400 may be further configured to determine if the reading pattern of the occupant 10 satisfies a predetermined standard corresponding to a proper reading of the textual information 40. In other words, it is determined if the driver 10 has actually performed a satisfactory enough task of reading the text or has simply skimmed through the text 40 or has been distracted by other ambient cues during performing the reading task.

In several example implementations, the impairment detection system 400 may be configured to determine the characteristic sequence of eye-movement parameters included in the gaze pattern profile by determining a first subset of metrics. The first subset of metrics comprise global eye movement measures including an eye fixation duration, an eye fixation rate, an eye saccade duration, an eye saccade rate, an eye saccade amplitude, and an eye saccade peak velocity for one or both eyes of the occupant 10. The global measures primarily indicate overall reading behavior and differences in text processing by the occupant 10. Generally, reading under the influence leads to increased fixation durations and a higher number of fixations. For example, under the influence of alcohol (average BAC ≈ 1%o), occupants may exhibit longer fixation durations, reduced fixation and saccade rates, and saccades that are shorter in duration, smaller in amplitude, and slower in peak velocity.

In some example implementations, for each eye tracking measure that is extracted, a mean value for each reading event may be calculated. In some examples, the impairment detection system 400 may present multiple instances of textual information to the occupant 10, e.g., by repeating display of similar or different text strings included in the first textual information 40, or make use of the circumstantial text that the occupant reads e.g. text present on traffic signs, etc. Accordingly, a mean value may be calculated for each extracted eye measure for each reading event by the system 400, and a final average value for each measure may be calculated afterwards across all the reading events.

In some example implementation, the impairment detection system 400 may further utilize dynamics eye movement measures. Accordingly, a second subset of eye movement measurement may include static entropy measurements of the eye fixation duration, the eye fixation position, the eye saccade duration, the eye saccade amplitude, the eye saccade peak velocity. Additionally or alternatively, the second subset of eye movement measurement may include transition entropy measurements of the eye fixation duration, the eye fixation position, the eye saccade duration, the eye saccade amplitude, the eye saccade peak velocity. Even further, the second subset of eye movement measurement may include a relationship between the eye fixation parameters such as eye fixation duration and parameters of the subsequent eye saccade measures.

Static and transition entropy in the present context is to be construed as two measures of dynamics of previously mentioned global fixation and saccade measures. In more detail, static entropy describes distribution of global eye movement measures in their value space; determining how equally the global eye movement measures are distributed in their values or in physical space. In contrast, transition entropy describes how equally the global eye movement measures change temporally.

In some example implementations, the impairment detection system 400, may further utilize word or sentence-level eye-movement measures, also referred to as local eye movement measures. Thus, a third subset of eye movement metrics may include a gaze regression, number of eye fixations and eye saccades per word comprised in the first textual information, the eye fixation position within a word comprised in the textual information e.g. in the first textual information, and the first eye fixation position within a word comprised in the textual information e.g. the first textual information. In the present context, gaze regression is to be understood as the eye saccade opposite to the normal reading direction. Thus, the measure is the eye saccade direction where the number of such eye saccades opposite to the normal reading direction are calculated. In the present context, the first eye fixation position within a word is compared to the word length, in order to determine its relations to the word. For example, if the first fixation along the length of a word lands, e.g., at 10% of the word length, this would mean that the first eye fixation position has landed at the beginning of the word. While if the first fixation along the length of the word lands, e.g., at 50% of the word length, this would mean that the first eye fixation position has landed in the middle of the word, and so on.

The local eye movement features might use calibrated or uncalibrated eye tracking data. In case the eye tracking data is uncalibrated, the eye tracking system 300 is adapted to automatically align recoded sensor data with the text 40 by selecting reading data using gaze pattern analysis and aligning that data so all fixations should fall within a defined margin of words and letters in the text 40.

Various suitable data analysis and statistics algorithms and ML or NN models may be used by the system 400 to analyze the recorded eye movement data. For instance, a one-way multivariate analysis of variance (MANOVA) may be used to determine the overall effect of intoxication on the combined extracted eye movement features. Furthermore, separate one-way analyses of variance (ANOVAs) may be used for each eye movement feature to determine the specific impact of intoxication on individual variables. Even further, in order to calculate probabilistic outcomes of the extracted features belonging to sober or intoxicated states, a logistic regression classifier models may be used to estimate discriminatory power of the extracted features by training the model on the standardized data of the extracted features. In should be clear that other suitable models or algorithms than the above-mentioned examples may be utilized for analyzing the gaze pattern profile of the occupants and determining the level and probability of impairment.

As mentioned previously, the calculated eye gaze features included in the gaze pattern profile are used as an input to an algorithm, which might be a threshold-based, or statistical algorithm or a ML or deep learning model. The algorithm estimates the impairment level and probability of the occupant 10 generating an impairment state value that may be presented on a scale of 0 to 1, or as a physical measurement, such as BAC or similar. As mentioned above, in example embodiments, each of the above extracted features such as fixation duration, or saccade amplitude, etc. may receive a respective probability of impairment score, and at a next step a final average probability of impairment score may be calculated based on values from all the extracted features.

As realized by the present inventor, employing global, dynamic, and local eye measures or any combination thereof during a natural reading event provides a robust and reliable metric for determining the impact of substances such as alcohol or drugs in order to predict the probability of impairment of the occupants 10. Accordingly, the impairment detection system 400 may apply the cascade of steps in determining the occupant impairment prior to allowing the occupant 10 such as the driver to start the engine of the vehicle 1 in order to use and operate i.e. drive the vehicle. In some example embodiments, the impairment detection system 400 may be implemented in a fleet of vehicles, such as a fleet of rental vehicles. Therefore, prior to driving off the vehicle 1 out of the rental center, the driver 10 of the vehicle 1 (or even the other passengers 10 for that matter) may undergo an effective sobriety and intoxication assessment while naturally interacting with instruments of the vehicle 1. This way a strong safety measure is imposed prior to driving the vehicle 1 in the traffic.

As mentioned earlier, the determined level and probability of the occupant impairment may be used on its own to prevent driving under influence, and/or may be provided as an input to other systems aboard the vehicle such as warning systems, or other impairment detection systems like DMS that are arranged to estimate impairment during driving, etc. In some example implementations, the recorded reading pattern of the occupants 10 may be used to assess if the driver 10 is ready to take over vehicle control from the ADS system 500 e.g. during an autonomous driving event.

In some example embodiments, the impairment detection system 400 may obtain a second set of measurements through the eye tracking system 300 and the plurality of imaging sensors 103 arranged on board the vehicle 1. The second set of measurements may capture at least one further parameter associated with the occupant 10 in addition to reading the textual information. For instance, the second set of measurements may be an eye blink duration of the occupant 10, an eye blink closing and opening velocity, an eye blink closing and opening duration, and a head pose and/or head movements of the occupant 10.
Accordingly, the impairment detection system 400 may be operable to determine the probability and the level of impairment of the occupant 10 further based on the obtained second set of measurements i.e. in addition to the characteristic sequences of the eye movement metrics as discussed above.

In several embodiments, upon determining that the probability and the level of impairment of the occupant falls within an acceptable range, the impairment detection system 400 may generate and transmit a control signal to any suitable entity of the vehicle 1 such as the control system 200 of the vehicle or the ADS system 500 in order to allow the driver 10 to take over control of the vehicle 1 and operate the vehicle 1. Thus, the control of the vehicle 1 will be extended to the driver 10 who will be able to either start the engine and the ignition system, or alternatively take over control from the ADS 500 during an autonomous driving event.

In contrast to scenarios that the occupants 10 like the driver 10 are determined as being at a sober state and capable of operating the vehicle 1, there will naturally be scenarios that the impairment detection system may determine that the occupant 10 is in the impaired state and not capable of operating the motor vehicle 1. According, in some example embodiments, upon determining that the probability and the level of impairment of the occupant 10 falls outside an acceptable range, the impairment detection system 400 may determine that the occupant 10 of the vehicle is in the impaired state. Thus, the impairment detection system 400 may be adapted to generate a control signal to be transmitted to a any suitable entity of the vehicle 1 such as the control system 200 of the vehicle, or the DMS systems, or the on-board ADS system 500. This control signal may control certain operations and functionalities of the vehicle 1 to ensure that the driver 10 is not allowed to operate the vehicle 1 in an intoxicated state. For instance, the signal may indicate the impairment of the driver 10 to another DMS system, to assist further investigations of the impaired state of the driver 10 prior to blocking the driver from assuming control of the vehicle 1. Additionally or alternatively, the control signal may be utilized by the impairment detection system 400 directly or indirectly through the control system 200 of the vehicle 1 to prevent the driver 10 from operating the vehicle 1. This may include functionalities such as deactivating an ignition system of the vehicle 1 and preventing the occupant 10 from turning on the ignition system prior to driving. Furthermore, it may include deactivating a gearbox or transmission system of the vehicle 1 to prevent the occupant 1 from driving the car, e.g. to allow the driver to start the engine and use other functions of the vehicle 1 in a stationary mode. Even further, the precautionary measures may include, when in an autonomous driving mode, preventing the ADS 500 of the vehicle from extending control of the vehicle 1 to the occupant 10. Furthermore, when in an autonomous driving mode, instructing the ADS 500 to bring the vehicle 1 to a stop, or instructing the ADS 500 to contact emergency services for assistance, or the like. In summary, the impairment detection system 400 will ensure, either prior to driving the vehicle 1, or during an active event that intoxicated drivers are prevented from unsafely operating the vehicle 1.

In some embodiments, the impairment detection system 400 may further be adapted to generate a second signal indicating the outcome of the determination of impairment of the occupant. In an event that the probability and level of impairment of the occupant 10 falls within the acceptable range, the system 400 may be further configured to prompt, by the second signal, the occupant 10 of the vehicle to trigger an interactive control element activated on the display module 101a, 402a. The interactive control element on the display module 101a, 402a may be a button such as the button 50 in Fig. 3, that is in its deactivated mode as shown by the dashed box 50a, and becomes activated and engageable as indicated by the box 50b upon determination of the sobriety of the occupant 10. The second signal may comprise any one of generating and displaying a second textual information 41 on the display module 101a, 402a indicative of the outcome of the determination process of the probability and the level of impairment of the occupant 10. If the occupant 10 is considered to be in an impaired state an example second text 41 may be "you are not in a condition to operate this vehicle" or similar statements informing the driver 10 of the situation. However, if the driver 10 is considered to be in the sober and capable state, activation of the previously deactivated interactive control element 50 on the display 101a, 420a may invite the driver 10 to press the button 50 and e.g. "Agree" or "consent" to some terms and conditions for using the vehicle. In some examples, the second signal may be a text message and/or a visual indicator presented on the display 101a, 402a and/or an auditory indicator directing the occupant 10 to trigger the interactive control element 50 or similar. In some example cases where evaluation of the driver impairment is mandatory before starting to drive the vehicle 1, the impairment detection system 400 may not allow the occupant 10 to proceed with using the vehicle 1 unless the driver 10 reads the second textual information 41 displayed and press the "Agree" (or similar) button, which may not be active until after the driver reads the second textual information 41. This effectively introduces an additional step to ensure that not only the driver is determined to be in the sober and capable state, but the driver 10 also willingly and consciously reads and agrees to the terms and conditions applicable to the use of the vehicle 1. The impairment detection system 400, may be adapted to continuously monitor the reading pattern of the occupant 10 during the time the occupant is reading the second textual information 41, and only allow the occupant to proceed if the reading pattern of the occupant 10 satisfies the predetermined reading patterns, to ensure that the driver 10 has properly read the second textual information 41. Thus, the impairment detection system 400 may only generate a respective control signal to (directly or indirectly through other entities such as the control system 200 of the vehicle 1) extend control of the vehicle 1 to the occupant 10 in order to operate the vehicle after the occupant of the vehicle has triggered the interactive control element 50 activated 50b on the display 101a, 402a as a result of occupant's gaze profile satisfying the predetermined reading pattern.

In some example implementations, the impairment detection system 400 may accordingly be configured to determine whether the generated gaze pattern profile for the occupant 10 satisfies a predetermined reading eye-movement pattern indicating that the occupant has properly read the displayed textual information 40. The predetermined reading eye-movement pattern may be a threshold-based assessment criteria including a specific sequence, timing, or order of eye fixations or saccades of a benchmark reading pattern. The comparison of the generated gaze profile and the predetermined benchmark will thus ensure that the eye movement data gathered during the reading activity by the occupant 10 poses the acceptable quality to be input to the impairment detection algorithms and models for establishing the impaired state of the occupant 10 more accurately. In some example implementations, upon determining that the generated gaze pattern profile satisfies the predetermined reading eye-movement pattern, the impairment detection system 400 may generate a third signal indicating that reading the textual information 40 has been performed properly by the occupant 10. The third signal may comprise a text message and/or a visual indicator presented on the display 101a, 402a and/or an auditory indicator indicating outcome of reading the textual information 40. For example, the display 101a, 402a may show a "thank you" message, may flash, or a voice command may indicate a message such as "thank you for your attention" or similar. Additionally, or alternatively, the third signal may be an activation of a previously deactivated interactive control element on the display 101a, 402a to be triggered by the occupant 10. For example, a deactivated "proceed" button may become activated after the occupant 10 satisfies the reading pattern requirements, so the driver 10 can press the button to move to the next step of assuming control of the vehicle. In some example embodiments, if it is determined that the reading pattern of the occupant does not satisfy the predetermined acceptable reading pattern criteria, the third signal may indicate this to the occupant. The indication may be in form an auditory or visual signal, or by displaying a text message inviting the driver to reread the textual information, or simply by displaying a different textual information or maintaining the previous textual information for the occupant to read.

The third signal may be comprised as a part of the second signal or may be an intermediate step prior to generating the second signal.

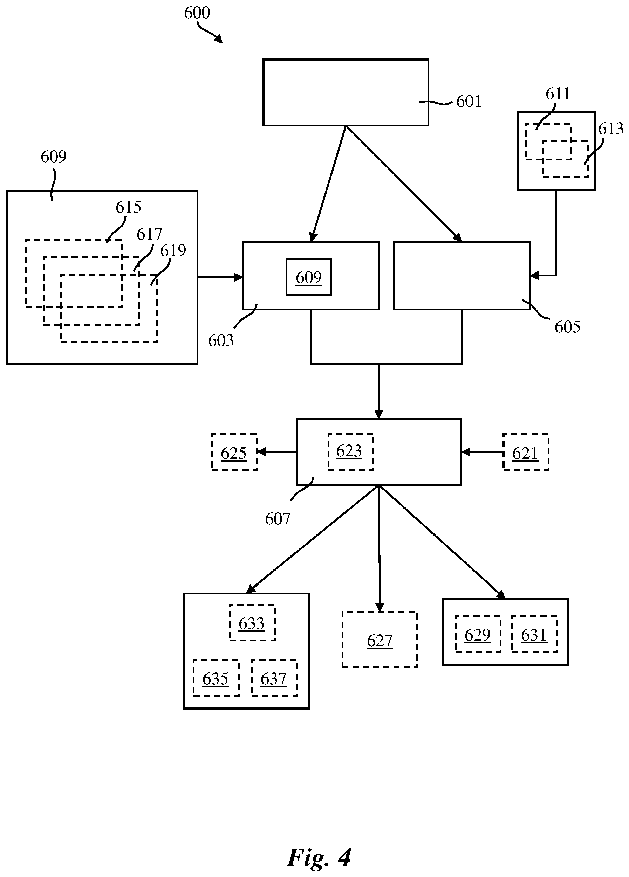

Fig. 4 shows a schematic flowchart of an exemplifying method **600** according to several embodiments of the present disclosure. The method **600** is a computer implemented method for predicting impairment of an occupant of a vehicle 1. In several embodiments, the method may be performed by the impairment detection system 400 in conjunction with the eye tracking system 300 and the control system 200 of the vehicle 1 as discussed previously. Accordingly, all features and advantages discussed with respect to the eye tracking system 300, the impairment detection system 400 and the control system of the vehicle 1 analogously apply to embodiments of the method **600** herein. The method **600** comprises obtaining **601,** by a plurality of imaging sensors 103 arranged on board the vehicle, a first set of measurements capturing one or more eye-movement patterns of the occupant 10. The method **600** further comprises generating **603,** based on the obtained first set of measurements, a gaze pattern profile for the occupant 10. In several embodiments the gaze pattern profile may be associated with reading textual information by the occupant 10. Accordingly, the method **600** further comprises determining **605** if the generated gaze pattern profile corresponds to an eye-movement pattern associated with reading textual information. When it is determined that the generated gaze pattern profile corresponds to the eye-movement pattern associated with reading textual information, the method **600** further comprises determining **607,** based at least partly on the generated gaze pattern corresponding to the eye-movement pattern associated with reading textual information, a probability and the level of impairment of the occupant. Generating **603** the gaze pattern profile for the occupant 10 comprises determining **609** a characteristic sequence of eye-movement metrics including a sequence of eye fixations and/or a sequence of eye saccades of one or both eyes of the occupant 10.

In some embodiments, the method **600** may further comprise displaying **611** a first textual information 40 on a display module 101a, 402a arranged on board the vehicle 1 with which the occupant interacts. The method may further comprise prompting 613, by a first signal, the occupant 10 of the vehicle to read the displayed first textual information. In some embodiments, the first signal may comprise any one of appearance of the textual information 40 on the display, and an instruction text included in the first textual information 40 encouraging the occupant to read the first textual information. The first signal may further comprise a visual and/or an auditory indicator directing attention of the occupant 10 to read the displayed textual information 40.

In some embodiments, the step of determining **609** the characteristic sequence of eye-movement parameters included in the gaze pattern profile may comprise determining **615** a first subset of metrics including an eye fixation duration, an eye fixation rate, an eye saccade duration, an eye saccade rate, an eye saccade amplitude, and an eye saccade peak velocity for one or both eyes of the occupant 10.

In some embodiments, the step of determining **609** the characteristic sequence of eye-movement parameters included in the gaze pattern profile may further comprise determining **617** a second subset of metrics including any one of static entropy measurements and/or transition entropy measurements of the eye fixation duration, the eye fixation position, the eye saccade duration, the eye saccade amplitude, the eye saccade peak velocity. The second subset of metrics may further include a relationship between the eye fixation duration and parameters of the subsequent eye saccade.

In some embodiments, the step of determining **609** the characteristic sequence of eye-movement parameters included in the gaze pattern profile may further comprise determining **619** a third subset of metrics including any one of a gaze regression, number of eye fixations and eye saccades per word comprised in the textual information e.g. the first textual information 40, the eye fixation position within a word comprised in the textual information e.g. the first textual information 40, and the first eye fixation position within a word comprised in the textual information.

In some exemplary embodiments, the method **600** may further comprise obtaining **621,** by the plurality of imaging sensors 103 arranged on board the vehicle, a second set of measurements capturing at least one parameter associated with the occupant 1. The second set of measurements may be obtained during an event or routine of reading the textual information by the occupant 10. The second set of measurements may comprise any one of an eye blink duration, an eye blink closing and opening velocity, an eye blink closing and opening duration, and a head pose and/or head movements of the occupant. The method **600** may further comprise determining **623** the probability and the level of impairment of the occupant based further on the obtained second set of measurements.

As mentioned earlier the vehicle 1 may comprise an ADS system 500 and an on-board DMS system. Accordingly in some embodiments, the method **600** may further comprise providing **625** the determined probability and the level of impairment of the occupant 10 to the DMS and/or to the ADS 500.

In several example embodiments, the method **600** may further comprise upon determining that the probability and the level of impairment of the occupant falls within an acceptable range, generating **627** a control signal in order to extend control of the vehicle 1 to the occupant 10 in order to operate the vehicle.

In some embodiments, the method **600** may further comprise upon determining that the probability and the level of impairment of the occupant falls outside an acceptable range such as a threshold value, determining **629** that the occupant of the vehicle is in an impaired state. The method **600** may further comprise generating **631** a control signal in order to perform any one of deactivating an ignition system of the vehicle and preventing the occupant from turning on the ignition system, deactivating the transmission system of the vehicle, when in an autonomous driving mode, preventing an automated driving system, ADS, 500 comprised in of the vehicle from extending control of the vehicle to the occupant, when in an autonomous driving mode, instructing the ADS 500 to bring the vehicle to a stop, and instructing the ADS 500 to contact emergency services for assistance.

In some example embodiments, the method **600** may further comprise generating **633** a second signal indicating an outcome of the determination of impairment of the occupant. When the probability and level of impairment of the occupant falls within the acceptable range, the method **600** may further comprise prompting **635,** by the second signal, the occupant 10 of the vehicle 1 to trigger an interactive control element 50 activated on the display 101a, 402a. The second signal may comprise any one of generating and displaying a second textual information 41 on the display module 101a, 402a indicative of the outcome of the determination process of the probability and the level of impairment of the occupant. The second signal may further comprise an activation of a previously deactivated interactive control element 50 on the display 101a, 402a. The second signal may further comprise a text message and/or a visual indicator presented on the display and/or an auditory indicator directing the occupant to trigger the interactive control element 50.

In some exemplary embodiments, the method **600** may further comprise extending **637** control of the vehicle 1 to the occupant 10 in order to operate the vehicle after the occupant of the vehicle has triggered the interactive control element 50 activated on the display.

The present disclosure has been presented above with reference to specific embodiments. However, other embodiments and example implementations than the above described are possible and fall within the scope of the disclosure and appended claims. According to exemplary embodiments, there is provided a non-transitory computer-readable storage medium storing one or more programs configured to be executed by one or more processors of a vehicle control system 200 and/or by one or more processors of the eye tracking system 300 and/or by one or more processors of the impairment detection system 400. The one or more programs comprising instructions for performing the method **600** according to any one of the above-discussed embodiments.

In several aspects and embodiments, there is provided a computer program product comprising instructions which, when the program is executed by one or more processors of a processing system, causes the processing system to carry out the method according to any one of the embodiments of the method of the present disclosure. The processing system may be any one of or a combination of the vehicle control system 200, the eye tracking system 300 and the impairment detection system 400.

Alternatively, or additionally according to some exemplary embodiments a cloud computing system can be configured to perform any of the methods presented herein. The cloud computing system may comprise distributed cloud computing resources that jointly perform the methods presented herein under control of one or more computer program products. Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The one or more processor(s) 201a, 301a, 401a associated with the control system 200, the impairment detections system 400 and the eye tracking system 300 may be or include any number of hardware components for conducting data or signal processing or for executing computer code stored in operatively coupled memory devices (not shown). The memory may be one or more devices for storing data and/or computer code for completing or facilitating the various methods described in the present disclosure. The memory may include volatile memory or non-volatile memory. The memory may include database components, object code components, script components, or any other type of information structure for supporting the various activities and functions of the present disclosure. According to some exemplary embodiments the memory is communicably connected to the one or more processors 201a, 301a, 401a (e.g., via a circuit or any other wired, wireless, or network connection) and includes computer code for executing one or more methods described herein.

It should be appreciated that the vehicle 1 may also comprise a communication/antenna interface which may further provide the possibility to send output to a remote location (e.g. remote operator or control centers) and the cloud network 20. The sensors such as imaging sensors 103 in the vehicle may communicate with the various processing systems 200, 300, 400 using a local network setup, such as CAN bus, I2C, Ethernet, optical fibers, wireless links and so on. Local communication within the vehicle may also be of a wireless type with protocols such as WiFi, LoRa, Zigbee, Bluetooth, or similar mid/short range technologies.

In some examples, the ML or NN algorithms and models may be implemented in the one or more processors 201a, 301a, 401a. In some examples, sensor data may be sent to an external system, wherein the external system comprises the ML algorithm or data analysis algorithms configured for processing recorded eye movements and extracting gaze features. It should be appreciated that different features and steps of the embodiments herein may be combined in other combinations than those described in the example figures.

As used herein, the term "if" may be construed to mean "when or "upon" or "in response to determining or "in response to detecting" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an "event" depending on the context. The term "obtaining" herein is to be interpreted broadly and encompasses receiving, retrieving, collecting, acquiring, and so forth directly and/or indirectly between two entities configured to be in communication with each other or with other external entities.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the disclosure. Likewise, in some cases some of the software implementations may be accomplished with standard programming techniques with rule-based logic and other logic to accomplish the various connection steps, processing steps, comparison steps and decision steps. The above-mentioned and described embodiments are only given as examples and should not be limiting to the present disclosure. Other solutions, uses, objectives, and functions within the scope of the disclosure as claimed in the below described patent embodiments should be apparent for the person skilled in the art.

## Claims

1. A computer implemented method of predicting impairment of an occupant of a vehicle, the method comprising:
obtaining, by a plurality of imaging sensors arranged on board the vehicle, a first set of measurements capturing one or more eye-movement patterns of the occupant; generating, based on the obtained first set of measurements, a gaze pattern profile for the occupant;
determining if the generated gaze pattern profile corresponds to an eye-movement pattern associated with reading textual information; and
upon said determination:
determining, based at least partly on the generated gaze pattern corresponding to the eye-movement pattern associated with reading textual information, a probability and the level of impairment of the occupant;
wherein the generating the gaze pattern profile comprises determining a characteristic sequence of eye-movement metrics including a sequence of eye fixations and/or a sequence of eye saccades of one or both eyes of the occupant.

2. The method according to claim 1, wherein the method further comprises:
displaying a first textual information on a display module arranged on board the vehicle with which the occupant interacts;
prompting, by a first signal, the occupant of the vehicle to read the displayed first textual information;
wherein the first signal comprises any one of appearance of the textual information on the display, an instruction text included in the first textual information encouraging the occupant to read the first textual information, and a visual and/or an auditory indicator directing attention of the occupant to read the displayed textual information.

3. The method according to any one of claims 1 or 2, wherein determining the characteristic sequence of eye-movement parameters included in the gaze pattern profile comprises determining a first subset of metrics including:
an eye fixation duration, an eye fixation rate, an eye saccade duration, an eye saccade rate, an eye saccade amplitude, and an eye saccade peak velocity for one or both eyes of the occupant.

4. The method according to claim 3, wherein determining the characteristic sequence of eye-movement parameters included in the gaze pattern profile further comprises determining a second subset of metrics including any one of:
static entropy measurements and/or transition entropy measurements of:
the eye fixation duration, the eye fixation position, the eye saccade duration, the eye saccade amplitude, the eye saccade peak velocity; and
a relationship between the eye fixation duration and parameters of the subsequent eye saccade.

5. The method according to any one of claims 3 or 4, wherein determining the characteristic sequence of eye-movement parameters included in the gaze pattern profile further comprises determining a third subset of metrics including any one of:
a gaze regression, number of eye fixations and eye saccades per word comprised in the textual information, the eye fixation position within a word comprised in the textual information, and the first eye fixation position within a word comprised in the textual information.

6. The method according to any one of the preceding claims, wherein the method further comprises:
obtaining, by the plurality of imaging sensors arranged on board the vehicle, a second set of measurements capturing at least one parameter associated with the occupant comprising any one of:
an eye blink duration, an eye blink closing and opening velocity, an eye blink closing and opening duration, and a head pose and/or head movements of the occupant;
wherein the method further comprises:
determining the probability and the level of impairment of the occupant based further on the obtained second set of measurements.

7. The method according to any one of the preceding claims, wherein the vehicle comprises an automated driving system, ADS, and an on-board driver monitoring system, DMS, and wherein the method further comprises:
providing the determined probability and the level of impairment of the occupant to the DMS and/or to the ADS.

8. The method according to any one of the preceding claims, wherein the method further comprises:
upon determining that the probability and the level of impairment of the occupant falls within an acceptable range:
generating a control signal in order to extend control of the vehicle to the occupant in order to operate the vehicle.

9. The method according to any one of claims 1 - 7, wherein the method further comprises:
upon determining that the probability and the level of impairment of the occupant falls outside an acceptable range:
determining that the occupant of the vehicle is in an impaired state;
generating a control signal in order to perform any one of:
deactivating an ignition system of the vehicle and preventing the occupant from turning on the ignition system, deactivating the transmission system of the vehicle, when in an autonomous driving mode, preventing an automated driving system, ADS, comprised in the vehicle from extending control of the vehicle to the occupant, when in an autonomous driving mode, instructing the ADS to bring the vehicle to a stop, and instructing the ADS to contact emergency services for assistance.

10. The method according to any one of claims 8 or 9, wherein the method further comprises:
generating a second signal indicating an outcome of the determination of impairment of the occupant; and when the probability and level of impairment of the occupant falls within the acceptable range:
prompting, by the second signal, the occupant of the vehicle to trigger an interactive control element activated on the display;
wherein the second signal comprises any one of:
generating and displaying a second textual information on the display module indicative of the outcome of the determination process of the probability and the level of impairment of the occupant,
an activation of a previously deactivated interactive control element on the display, a text message and/or a visual indicator presented on the display and/or an auditory indicator directing the occupant to trigger the interactive control element.

11. The method according to claim 10, wherein the method further comprises:
extending control of the vehicle to the occupant in order to operate the vehicle after the occupant of the vehicle has triggered the interactive control element activated on the display.

12. A computer program product comprising instructions which, when the program is executed by one or more processors of a processing system, causes the processing system, to carry out the methods according to any one of the preceding claims.

13. An impairment detection system for predicting impairment of an occupant of a vehicle, the system comprising a controller module comprising one or more processors configured to:
obtain, by a plurality of imaging sensors arranged on board the vehicle, a first set of measurements capturing one or more eye-movement patterns of the occupant;
generate, based on the obtained first set of measurements, a gaze pattern profile for the occupant;
determine if the generated gaze pattern profile corresponds to an eye-movement pattern associated with reading textual information; and upon said determination, the controller module is further configured to:
determine, based at least partly on the generated gaze pattern corresponding to the eye-movement pattern associated with reading textual information, a probability and the level of impairment of the occupant;
wherein the controller module is further configured to generate the gaze pattern profile by determining a characteristic sequence of eye-movement metrics including a sequence of eye fixations and/or a sequence of eye saccades of one or both eyes of the occupant.

14. The impairment detection system according to claim 13, wherein the controller module is further configured to:
display a first textual information on a display module arranged on board the vehicle with which the occupant interacts; and
prompt, by a first signal, the occupant of the vehicle to read the displayed first textual information;
wherein the first signal comprises any one of appearance of the textual information on the display, an instruction text included in the first textual information encouraging the occupant to read the first textual information, and a visual and/or an auditory indicator directing attention of the occupant to read the displayed textual information.

15. The impairment detection system according to any one of claims 13 or 14, wherein the controller module is further configured to:
determine characteristic sequence of eye-movement metrics included in the gaze pattern profile by determining at least a first subset of metrics including an eye fixation duration, an eye fixation rate, an eye saccade duration, an eye saccade rate, an eye saccade amplitude, and an eye saccade peak velocity for one or both eyes of the occupant.
